# EUROPEAN PATENT APPLICATION

(11) **EP 4 016 369 A1**
(43) Date of publication of application: **22.06.2022**
(21) Application number: 20214291.5
(22) Date of filing: 15.12.2020
(51) Int. Cl.: G06F 40/157, G06F 40/242, G06F 40/30, G06N 20/00, G10L 15/00, H03M 7/30

(54) **GENERATING ENCODED DATA**

(71) Applicant: Koninklijke Philips N.V., 5656 AG Eindhoven (NL)
(72) Inventor: VAN DEN DUNGEN, Wilhelmus Andreas Marinus Arnoldus Maria, 5656 AE Eindhoven (NL)
(74) Representative: Philips Intellectual Property & Standards

(57) **Abstract**

In an embodiment, an apparatus (100) is described. The apparatus comprises processing circuitry (102), which comprises a recognition module (104), a generating module (106) and a transmitting module (108). The recognition module is configured to implement a natural language processing, NLP, model configured to recognize, in a user data sample, a term from a vocabulary associated with a personal care regime. The generating module is configured to generate encoded data indicative of the term, wherein the encoded data uses less memory than the user data sample. The transmitting module is configured to transmit the encoded data.

## Description

### FIELD OF THE INVENTION

The invention relates to a method, apparatus and tangible machine-readable medium for communicating data.

### BACKGROUND OF THE INVENTION

Personal care devices such as grooming devices and oral healthcare (OHC) devices, and certain Internet of Things (IoT) devices, may utilize wireless communication technologies in order to facilitate a 'smart' or enhanced user experience via a data connection to a data processing system such as a local, or 'edge', device or in the cloud. Data from a device such as an audio interface, display interface and/or sensor may be used to complement the user experience, for example, by allowing the user to interact with the device using audio data, visual data and/or sensor data to facilitate exchange of information as part of the user experience.

Such user experiences may lead to a large amount of data being transferred over the data connection, which may lead to an increased cost. A personal care device having a user-friendly form factor may implement various wireless communication technologies such as Bluetooth^{™} Low Energy (BLE), Wi-Fi, Cellular (e.g., 3G, 4G, 5G, etc), Narrowband Internet of Things (NB-IoT), Long Range (LoRa) wireless communication, Long Term Evolution Machine Type Communication (LTE-M), etc. in order to provide the user with access to a user service, for example, provided by a data processing system in the cloud via a data connection.

There are some scenarios where a personal care device or an IoT device may rely on another UE (e.g., a UE such as a mobile phone, another IoT device and/or a base station such as a Wi-Fi hub) for facilitating data communication with a data processing system such as in the cloud. However, if the other UE is out of range or the data communication service is not working, the 'smart' functionality of the personal care/IoT device may be limited. Further, in some cases, the other UE may be expensive, complex, large, involve a high operational cost and/or have constraints in terms of how much power can be used. Further, a data service may have high fees for enabling data services and/or may have constraints in terms of how much bandwidth is available. In the case of high bandwidth channels, these channels may not be used all of the time and yet a fee may be paid for the service. Further still, an internet-connected device may utilize complex/high cost chips to facilitate the data communication. Further the device may use other high cost components and/or have to meet an appropriate design specification for the device to ensure compatibility with certain antenna technologies and/or other constraints associated with the chips used for data communication in the device.

Connected technologies may collect data from various sources to evaluate a user experience. For example, a personal care device may comprise various sensors to monitor the performance of the device. Examples of sensors include motion sensors such as accelerometers, current monitoring in motor drives, optical and temperature sensing, etc. A cloud-based service may provide some feedback to the user based on certain data collected. However, the data collected may not be understood sufficiently to complement the user experience.

### SUMMARY OF THE INVENTION

Aspects or embodiments described herein may relate to reducing communication resource usage in certain settings involving use of a personal care device as part of a personal care regime. Aspects or embodiments described herein may obviate one or more problems associated with providing a service for a user of a personal care device with certain constraints such as relating to the availability of a communication resource for facilitating service provision and/or constraints associated with device design.

In a first aspect, apparatus is described. The apparatus comprises processing circuitry. The processing circuitry comprises a recognition module, generating module and a transmitting module. The recognition module is configured to implement a natural language processing, NLP, model. The NLP model is configured to recognize, in a user data sample, a term from a vocabulary associated with a personal care regime. The user data sample comprises a natural language representation of the term. The generating module is configured to generate encoded data indicative of the term. The encoded data uses less memory than the user data sample. The transmitting module is configured to transmit the encoded data.

In some embodiments, the vocabulary associated with the personal care regime comprises a plurality of terms associated with user interaction with a personal care device for assisting a user with their personal care regime.

In some embodiments, the NLP model is implemented by an artificial intelligence, AI, model trained to recognize, in the user data sample, the term from the vocabulary associated with the personal care regime.

In some embodiments, the AI-implemented NLP model is trained using user training data derived from a plurality of users' interaction with a specified type of personal care device associated with a specified type of personal care regime.

In some embodiments, the recognition module is configured to train and/or update the NLP model using the user data sample and/or a sensor measurement associated with a user's personal care regime.

In some embodiments, the user data sample is in an audio data format, and wherein generated encoded data indicative of the recognized term uses less memory than the user data sample comprising the natural language representation of the recognized term.

In some embodiments, the apparatus comprises a memory storing a mapping between at least one term of the vocabulary and corresponding encoded data representative of the at least one term. In response to recognizing the term in the user data sample, the generating module is configured to access the memory to generate the encoded data corresponding to the recognized term.

In some embodiments, the processing circuitry comprises a receiving module configured to receive, from another entity, information for implementing functionality of the recognition module and/or generating module.

In some embodiments, the information is received in response to a request sent by the apparatus to the other entity. The request comprises an indication of unrecognizable information in the user data sample and/or a sensor measurement. The information received in response to the request comprises: an update for the NLP model; at least one term, recognized by the other entity, in the user data sample; and/or contextual information associated with the at least one term recognized by the other entity and/or the sensor measurement.

In some embodiments, the transmitting module is configured to transmit the request in response to the recognition module being unable to recognize at least part of the user data sample and/or interpret the sensor measurement.

In some embodiments, the received information comprises a response statement generated in response to the term indicated by the encoded data transmitted by the transmitting module. The response statement is in the same data format as the encoded data. The processing circuitry further comprises a conversion module. The conversion module is configured to convert the response statement to a corresponding natural language representation of the term based on a mapping between the response statement and construction information for constructing the natural language representation of the term.

In some embodiments, the recognition module is configured to interpret the recognized term from the vocabulary associated with the personal care regime and/or a sensor measurement associated with a user's personal care regime based on a protocol stored in a memory accessible to the recognition module. The protocol specifies a reaction to the interpreted term and/or sensor measurement. The apparatus is configured to cause a user interface to present the reaction to the user.

In some embodiments, the processing circuitry comprises a dialog management module. The dialog management module is configured to receive an indication of the recognized term and/or contextual information associated with a user experience. The dialog management module is further configured to generate a response to the indication based on a protocol. The response comprises information for updating the NLP model and/or a request for further information to be collected from the user in accordance with the protocol.

In a second aspect, a method is described. The method is a computer-implemented method. The method comprises recognizing, using a natural language processing, NLP, model, a term from a vocabulary associated with a personal care regime in a user data sample comprising a natural language representation of the term. The method further comprises generating encoded data indicative of the term, wherein the encoded data uses less memory than the user data sample. The method further comprises transmitting the encoded data.

In a third aspect, a tangible machine-readable medium is described. The tangible machine-readable medium stores instructions which, when executed by at least one processor, cause the at least one processor to implement the method according to the second aspect.

These and other aspects of the invention will be apparent from and elucidated with reference to the embodiment(s) described hereinafter.

### BRIEF DESCRIPTION OF THE DRAWINGS

Exemplary embodiments of the invention will now be described, by way of example only, with reference to the following drawings, in which:
Fig. 1 is a schematic drawing of an apparatus for communicating data according to an embodiment;
Fig. 2 is a schematic drawing of a system for communicating data according to an embodiment;
Fig. 3 is a schematic drawing of a system for training a model according to an embodiment;
Fig. 4 is a schematic drawing of an apparatus for communicating data according to an embodiment;
Fig. 5 is a schematic drawing of a system for implementing certain embodiments;
Fig. 6 refers to a method of communicating data according to an embodiment;
Fig. 7 is a schematic drawing of a machine-readable medium for communicating data according to an embodiment;
Fig. 8 is schematic drawing of various systems for implementing certain embodiments;
Fig. 9 is a schematic drawing of an apparatus for improving user experience;
Fig. 10 is a schematic drawing of an apparatus for improving user experience;
Fig. 11 refers to a method of improving a user experience;
Fig. 12 is a schematic drawing of a machine-readable medium for improving user experience; and
Fig. 13 is a schematic drawing of a system for implementing certain apparatus, methods and machine-readable media.

### DETAILED DESCRIPTION OF THE EMBODIMENTS

A user equipment (UE) may refer to a computing device in the vicinity of the user for performing certain tasks. Certain UEs such as a cellular phone, base station (e.g., Wi-Fi hub) and some IoT devices may perform tasks such as data collection, data processing and/or data relaying at or near a network edge (i.e., not in the cloud). Certain UEs may be referred to as 'edge devices' since they can connect to a network (e.g., the cloud), for example, using a cellular service or a wired internet connection. Thus, UEs such as cellular phones, base stations and some IoT devices may be regarded as 'edge devices'. Certain UEs may communicate with other UEs, for example, using device-to-device communication. Certain UEs such as personal care devices, as described in more detail below, may perform certain tasks associated with a personal care regime. Some personal care devices may not be directly connectable to the network, in which case, such personal care devices may not be regarding as 'edge devices'. However, some personal care devices may be directly connectable to the network, including via another UE such as a base station, and may therefore be regarded as an 'edge device'. Accordingly, UEs described here may refer to any device associated with a user of a personal care device, whether or not such a device may be connected to the network.

As will be described in more detail herein, a UE such as a personal care device, base station and/or other device in the vicinity of the user may implement certain methods, machine readable media and apparatus described herein. For example, the UE may comprise processing circuitry for implementing such methods, machine readable media and apparatus. The UE may be configured to provide improved data communication and/or an improved user experience in certain settings.

Examples of personal care devices include grooming devices (e.g., grooming devices such as shavers, epilators, hair styling devices, etc.) and oral healthcare devices (OHC) (e.g., electric toothbrushes, etc).

Personal care devices may be used for a personal care regime where the user may use the personal care device for their beauty or healthcare regime, or some other self-care regime, which may involve a regular, frequent or repeated use of the personal care device. In other similar words, the personal care device may be used on a regular basis (e.g., at least once a day, or several times over a period of time such as a week, month or year) as part of a beauty or healthcare regime, or some other self-care regime. In some examples, a personal care device may refer to a personal care regime device.
Examples of base stations include a user's home Wi-Fi hub, a smart phone (e.g., for relaying communications between the personal care device and a data processing system such as in the cloud), etc.

### Communication Resource Use

A low data rate communication channel may not be able to support certain user experiences, for example, where a certain amount of data is to be transmitted within a specified time window. Where a user experience relies on communicating a large amount of data to/from a data processing system in the cloud, the user may experience lag, which may degrade the overall experience unless the user can use a high bandwidth connection. However, this scenario may imply the need to use a high bandwidth cellular subscription or other network subscription with associated data costs. Further, the UE (e.g., the personal care device, base station or other UE) may need to incorporate complex, high-cost, high power-consumption technology such as a specific chip to access the network, which may have further cost and form-factor implications.

Some user experiences may involve audio communication, for example, to enable a user to interact with a UE such as a personal care device and/or edge device. Such interaction may comprise recording and/or interpreting (e.g., natural language in) the user's speech, playing audio data such as information for the user in audio format. The audio data may comprise a natural language representation of the user's speech or other audio information. The audio communication protocol may use a high bandwidth data connection in order to provide sufficient communication quality for the user experience, for example, to avoid corruption of the audio data so that it can be understood by the user and/or an application interpreting the audio data. In one scenario, audio communication involves transferring live audio data from a UE to the cloud (e.g., for processing to determine a response and/or for facilitating a human response to the audio). The response may be sent back to the UE via a data channel, which may be a high bandwidth channel for transferring audio data.

Some user experiences may involve a formatted text communication, for example, to enable a user to interact with the UE (e.g., by receiving user input such as text input and/or by displaying formatted text). The formatted text may comprise a natural language representation of the user input. The formatted text communication protocol may use a sufficiently high bandwidth data connection in order to provide sufficient communication quality for transmitting the formatted text.

The above user experiences may imply certain costs such as cloud-data storage and processing costs, the cost of providing the networked connection for such UEs, certain hardware/software costs due to providing a user-friendly device/proposition capable of high-end audio communication (and/or for displaying formatted text) and/or a high operational cost for accessing the networked connection. Although a low bandwidth connection could be used for communicating audio and/or formatted text data, the data transmitted by such a connection may be of insufficient quality for adequately complementing the user experience.

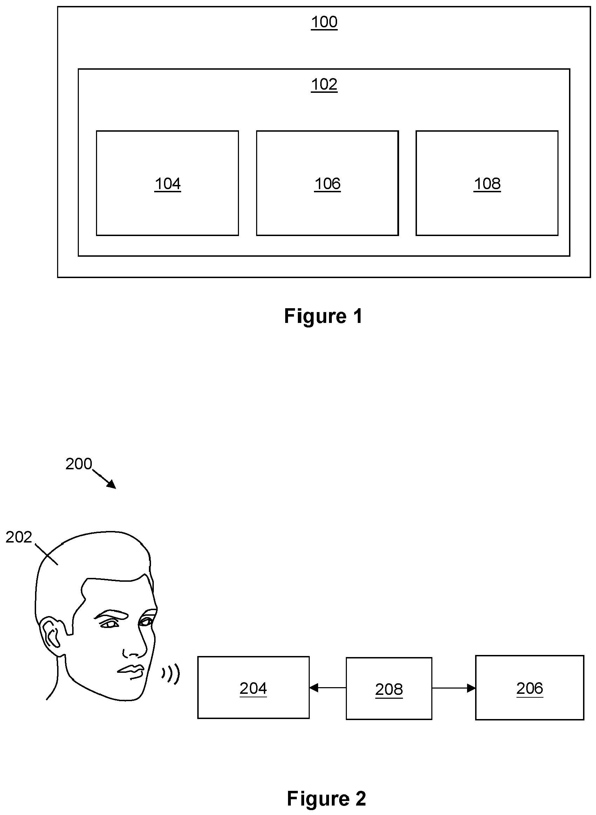

Fig. 1 shows an apparatus 100 for communicating data. The apparatus 100 may improve data communication and/or user experience in certain settings involving use of a personal care device. In this embodiment, the apparatus 100 is used to generate encoded data, which may be regarded as an example of compressed data capable of conveying high quality audio data or another data type such as formatted text with a relatively low amount of memory/communication resource needed to store/communicate the encoded data.

The apparatus 100 comprises processing circuitry 102 for implementing the modules below. In one example, the functionality of the apparatus 100 may be implemented by at least one processor executing instructions stored in a machine-readable medium, where such instructions correspond to the functionality of the blocks described below. In another example, the apparatus 100 may be implemented by at least one dedicated processor such as an Application Specific Integrated Circuit (ASIC), Field Programmable Gate Array (FPGA), Artificial Intelligence (AI) engine, etc (e.g., comprising dedicated processing circuitry and/or a memory storing instructions for causing the at least one dedicated processor to implement the functionality of the apparatus 100).

The apparatus 100 may be implemented by a UE such as a personal care device, base station or other UE.

In some embodiments, the personal care device may be directly connected to a data processing system such as in the cloud, for example, via a low data rate communication channel as described herein.

In some embodiments, another UE such as a base station may be directly connected to a data processing system such as in the cloud, for example, via a low data rate communication channel as described herein.

In some embodiments, the personal care device and the other UE may be connected to each other via a low data rate communication channel as described herein.

In some embodiments, at least one of: the UE and/or a cloud-based service may provide the data processing system (e.g., one or both of the UE and cloud may perform data processing, for example, depending on the availability of a communication resource and/or whether either of these entities has the relevant input for performing the data processing.

The processing circuitry 102 comprises a recognition module 104, a generating module and a transmitting module 108.

The recognition module 104 is configured to implement a natural language processing, NLP, model. The NLP model is configured to recognize, in a user data sample, a term from a vocabulary associated with a personal care regime. The user data sample comprises a natural language representation of the term.

In some embodiments, the NLP model is implemented using artificial intelligence (AI) and may comprise a neural network trained to recognize natural language in the user data sample. Such an AI-based NLP model may be trained in the cloud (e.g., at another entity such as a server) and/or at a UE (e.g., another device in the vicinity of the apparatus 100 and/or at the apparatus 100 itself). The training may be implemented using data from a plurality of users (e.g., including from a user of a personal care device) and may, in some examples, involve input from an expert or human trainer (e.g., annotation of the data). In some embodiments, the NLP model is implemented by an artificial intelligence, AI, model trained to recognize, in the user data sample, the term from the vocabulary associated with the personal care regime.

In some embodiments, which are not AI-implemented, the NLP model comprises a pre-determined set of rules (e.g., based on expert input and/or statistical analysis) for responding to a natural language input. In some cases, the vocabulary used in such an NLP model may be restricted to reduce processing time. In the present case, the vocabulary associated with the personal care regime may be restricted, for example, based on a specified 'type' of personal care device (e.g., a grooming device may be one 'type' of personal care device with an associated vocabulary and an oral healthcare device may be another 'type' of personal care device with a different associated vocabulary).

As referred to herein, certain embodiments involving the NLP model may or may not be AI-implemented. Where a reference is made to 'training' or 'learning' in association with the NLP model, this NLP model may also be referred to as an AI-implemented NLP model.

In use, a user may generate a user data sample, which is received by the apparatus 100 (e.g., directly received via a user interface or received from another UE that comprises a user interface) and the recognition module 104 may recognize the term from the vocabulary, if it is present in the user data sample.

In some embodiments, the 'user data sample' may refer to: an audio data sample comprising the natural language representation and/or a formatted text message comprising the natural language representation. For example, an audio data sample may comprise user speech comprising certain words spoken by the user, for example, when interacting with the personal care device (e.g., words spoken while shaving if the personal care device is a shaver). In another example, a formatted text message may comprise user input, for example entered via a user interface such as a touch-display, comprising a text such as a query or another interaction. In either case, the user data sample may comprise natural language, which can be understood by another human or the NLP model, when trained.

As mentioned above, the vocabulary is associated with a 'personal care regime' such as described above. Different regimes may have different words, or 'terms', associated with the use of a personal care device for use in a personal care regime.

In one example regime such as shaving, example terms from the vocabulary may include: 'skin', 'hair', 'face', 'stubble', 'shave', 'irritation', 'redness', etc.

In another example regime similar to shaving such as epilating, example terms from the vocabulary may include: 'skin', 'hair', 'legs', 'irritation', 'redness', etc. In this example, the vocabulary is slightly different to the shaving example even though there may be some terms which are the same. An epilator has different properties and may be used in a different way to a shaver. Accordingly, it may be anticipated that a user may have a different vocabulary when using these devices.

In another example regime such as tooth brushing, example terms from the vocabulary may include: 'teeth', 'gums', 'tongue', 'bleeding', 'whitening', 'tartar', 'plaque', etc.

For the above example regimes, it can be understood that the vocabularies used may depend on the type of regime and/or the type of personal care device for facilitating the regime.

In some embodiments, an AI-implemented NLP model is trained to recognize terms from a certain vocabulary associated with a certain type of personal care device associated with a certain personal care regime. The size of the AI-implemented NLP model and/or a database accessible to the apparatus 100 may be relatively small in terms of memory size depending on the size of the vocabulary range associated with use of the personal care device.

The construction of the NLP model and/or the database may be based on already available knowledge (e.g., a vocabulary from a plurality of users of a certain type of personal care device). In an example, the terms in the vocabulary may comprise a list of the most commonly used words during user interaction with a certain type of personal care device. These words in the vocabulary may be limited in number due to the limited range of vocabulary anticipated when a user interacts with the personal care device. In some cases, the user interaction may involve feedback from the personal care device and the words in the vocabulary may be focused on the feedback needed by the user. If the words are learned at the UE, another neural network could be trained to select the appropriate words for communication from the list.

The training of the NLP model may be implemented in the cloud or on a UE. In some cases, the training may move from the cloud to the UE (i.e., once the cloud has performed sufficient training). In some cases, the training may move from a first UE to the cloud or a second UE (i.e., if the first UE does not recognize certain information due to insufficient training). The apparatus 100 may therefore facilitate a UE-based implementation of an NLP model with the option to train the NLP model using the UE, or if needed, train the NLP model in the cloud.

Thus, in some embodiments, the vocabulary associated with the personal care regime comprises a plurality of terms associated with user interaction with a personal care device for assisting a user with their personal care regime.

In some embodiments, an AI-implemented NLP model is trained using user training data derived from a plurality of users' interaction with a specified type of personal care device associated with a specified type of personal care regime.

In some embodiments, the recognition module is configured to train and/or update the NLP model using the user data sample and/or a sensor measurement associated with a user's personal care regime. For example, the personal care device may comprise a sensor that takes a measurement or set of measurements. Such measurements may be associated with the user experience. For example, if the personal care device is a shaver and too much pressure is applied by the user, this may cause skin irritation or affect another shaving metric. Such pressure may be detected by an on-board sensor in the personal care device and the measurement fed to the apparatus 100 to facilitate training of the NLP model. Other embodiments described herein refer to further applications of the sensor measurement data for training and/or improving user experience. In some cases, another UE may comprise the sensor. In other words, the personal care device may not itself take the sensor measurement. An example scenario may be a camera positioned to acquire an image of the user while they are using the personal care device.

The generating module 106 is configured to generate encoded data indicative of the term, wherein the encoded data uses less memory than the user data sample.

In response to recognizing the term, the recognition module 104 may look up the term in a database accessible to the recognition module 104 and cause the generating module to generate the encoded data (e.g., from the database). For example, the generating module 106 may also have access to the database. The database may comprise the vocabulary associated with the personal care device and encoded data corresponding to each term in the vocabulary. The encoded data corresponding to the term may use a small amount of memory (e.g., of order of a byte). In this example, the encoded data may comprise a byte indicator or a codeword that represents the term.

The encoded data may use a relatively small amount of memory (e.g., one byte, or another small data size). The generated encoded data (e.g., comprising one byte of data) may therefore convey a relatively large amount of information compared to its data size (i.e., the byte of encoded data may contain the same amount of contextual information as an initial data sample comprising a natural language representation of the same data, which may otherwise use up a much larger amount of memory such as several Kilobytes or Megabytes, depending on the size and quality of the initial data sample).

In one example, a user may express that they have irritated skin through speech e.g., "my skin feels irritated". The recognition module 104 may recognize this speech in the user data sample (e.g., audio data) and cause the generating module 106 to look up the corresponding encoded data from the database and generate the encoded data. The database may comprise a plurality of terms of the vocabulary and each corresponding encoded data may be representative of a certain term of the vocabulary. If the encoded data has a memory size of one byte, this may be much smaller than the natural language representation of this data (e.g., in formatted text form or in audio data form). In some examples, the example expression "my skin feels irritated" in formatted text form may have a size of 23 bytes. In some examples, the same expression "my skin feels irritated" in audio data form may use many hundreds of bytes or kilobytes, depending on the quality and length of the user data sample.

A single byte indicator may represent the expression "my skin feels irritated", which may be a pre-determined byte indicator stored in the database. The single byte indicator may therefore use a much smaller memory size than the formatted text example or audio data sample.

The recognition module 104 may recognize contextual information in the user data sample. The recognition of the contextual information may be based on the most recent (e.g., live or current) user data sample and/or at least one previous user data sample. The recognition of the contextual information may be further based on the most recent (e.g., live or current) sensor measurement and/or at least one previous sensor measurement (i.e., where such sensor measurements are taken). For the example dialog, "my skin feels irritated", the NLP model may be trained to recognize the context of this expression due to the terms, 'skin' and/or 'irritated' being present in this user data sample. Since the vocabulary may contain these terms (e.g., where the vocabulary is associated with personal care regime of shaving or epilating), the recognition module 104 may cause the generating module 106 to generate the appropriate corresponding encoded data for these terms. In some examples, the encoded data could be a single byte representative of the context of the recognized term(s). For example, within a training data set (e.g., obtained from a plurality of users) associated with shaving or epilating, it may be relatively common for users to mention they have irritated skin. The NLP model may therefore be trained to recognize natural language that hints that the user may have irritated skin. The database may comprise byte indicators (or other encoded data) corresponding to any expected or common terms in the vocabulary.

In some embodiments, the user data sample is in an audio data format. In such embodiments, the generated encoded data is indicative of the recognized term and uses less memory than the user data sample comprising the natural language representation of the recognized term.

The transmitting module 108 is configured to transmit the encoded data. For example, the transmitting module 108 may be configured to transmit the encoded data to another entity such as another UE or the cloud via a communication channel (e.g., a low bandwidth channel or any communication channel that does not support or permit rapid communication of large amounts of data).

Thus, by generating and transmitting the encoded data (e.g., being representative of contextual information associated with the personal care regime), the apparatus 100 may support communication at a low-data rate and/or support communication of a relatively large amount of contextual information without having to communicate the information in its natural language form. Thus, the generated encoded data may convey a relatively large amount of information (i.e., when otherwise expressed in natural language form) within a relatively small data size (e.g., a 'byte indicator'), which may efficiently use or reduce the amount of resources (e.g., communication channel resource, hardware and/or software resource, etc) involved in communicating the information in the user data sample (e.g., to another entity such as a UE or the cloud). In other similar words, the apparatus 100 may understand the context of the information in the user data sample and cause the generating module 106 and transmitting module 108 to generate and transmit encoded data representative of the contextual information.

In some embodiments, the apparatus 100 may utilize natural language processing, e.g., via machine learning/AI to, based on a known/relatively restricted vocabulary range related to the proposition (e.g., personal care regime). The apparatus 100 may translate or covert the user data sample (e.g., user dialog) into a coded message sequence (e.g., comprising at least one byte indicator). By generating the encoded data, the apparatus 100 may reduce a communication channel resource use from a high bandwidth channel to a potentially low bandwidth channel and/or facilitate transmission of the encoded data at a very low bitrate. In some embodiments, the vocabulary and NLP AI engine can be trained on existing or captured data (e.g., from a plurality of users, which may include data from the present user) and the service facilitated by the NLP model can move from a fully cloud based connected solution (e.g., for initial training of the NLP model) to a mostly local processed solution at the UE (e.g., for personalized training of the NLP model). Such an approach may reduce the need for dedicated hardware/time for performing the initial training process at the UE.

In some embodiments, the apparatus 100 may facilitate low-data rate communication between UEs such as between a personal care device and a base station (such as a user's home Wi-Fi hub, phone or other UE). In some embodiments, the apparatus 100 may facilitate low-data rate communication between a UE such as a personal care device (or a base station) and a cloud-based service. Any combination of these embodiments may be facilitated by certain methods described herein.

In an example use scenario, the apparatus 100 may facilitate use of a narrow bandwidth communication channel and/or low data transfer between UEs due to the NLP model and the associated user data sample collected by one of these edge devices (e.g., voice prompts corresponding to a user data sample in an audio data format recorded by one of these devices).

Fig. 2 shows an example system 200 for communicating data to/from a user 202. The system 200 comprises a UE 204 implementing the functionality of certain apparatus described herein (e.g., the apparatus 100). The system 200 further comprises another entity 206 (e.g., another UE in the vicinity or a cloud-based service) for implementing certain functionality associated with training the NLP model used in the apparatus 100. A communication channel 208 between the UE 204 and the other entity 206 is used for communicating the data. As mentioned above, the apparatus 100 supported by the UE 204 may facilitate communication of a large amount of contextual information from the user 202 to the other entity 206 in the encoded data format, which may reduce the resources needed for conveying the context information. For example, the amount of memory used to convey the encoded data via the communication channel 208 may be less than a natural language representation of the user data sample (e.g., audio data and/or formatted text).

In some embodiments, the amount of bandwidth needed to be made available for the communication channel 208 may be smaller compared with a higher bandwidth technology such as cellular communication technologies like 3G, 4G, 5G, etc. Further, the UE 204 may involve a simpler hardware/software implementation for communicating data compared with some cellular devices, use a lower amount of power and/or involve a relatively inexpensive IoT communication technology such as BLE, NB-IoT, LoRa or Sigfox technologies. The combination of a relatively inexpensive/low bandwidth IoT communication technology (e.g., implemented by the UE 204 comprising the apparatus 100) with a higher bandwidth technology in certain other UEs (e.g., a base station or mobile phone) may facilitate a robust, small, effective handheld audio enabled system. For example, the UE 204 with the relatively inexpensive communication technology may communicate with another UE such as a mobile phone, which may be able to communicate at a high data rate with a cloud-based service. However, the UE 204 may not need to include certain hardware/software to facilitate communication with a high bandwidth technology, which may otherwise make the UE 204 expensive to implement, need to incorporate more hardware into the overall package, etc.

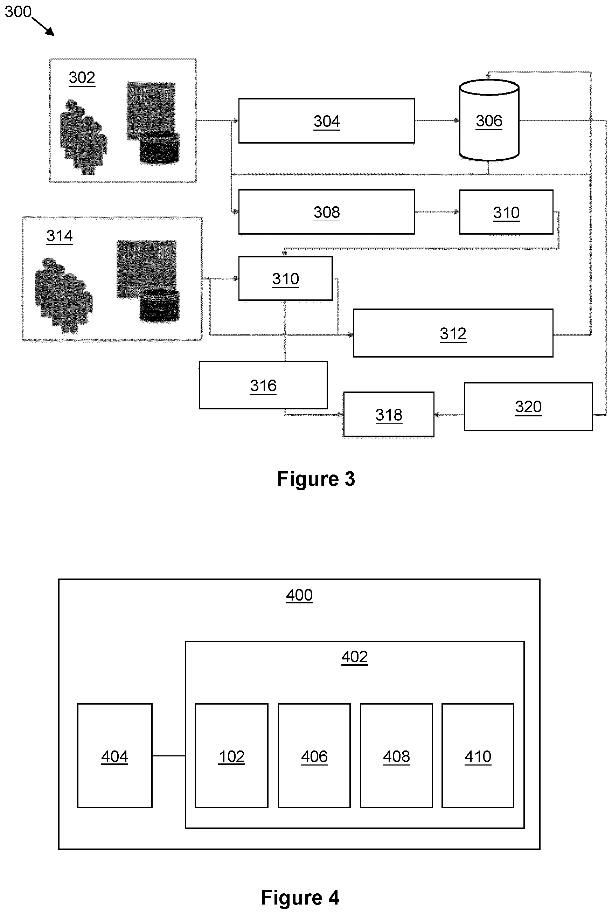

Fig. 3 shows an example system 300 for training the NLP model described in relation to the apparatus 100, with reference also to the system 200 of Fig. 2.

In an example training scenario, a service provider may determine the limited vocabulary set associated with the personal care regime type (e.g., male grooming, oral healthcare, etc) by analyzing at least one existing protocol associated with the personal care regime type, as well as possible audio recordings and/or user text messages associated with the personal care regime (e.g., from a plurality of users). An algorithm may identify certain words for the data training set (e.g. common words used by a user when interacting with a specified type of personal care device). The training data set may be created to train a neural network to respond and communicate using and combining the relatively restricted vocabulary set.

In some embodiments, once trained, the NLP model may be implemented in a UE and/or in the cloud in combination with the vocabulary terms implemented as auto-selectable and/or user voice prompts. In some cases, such prompts may be triggered by an event such as a sensor measurement and/or algorithm detecting a potential issue or risk in connection with the user and/or their personal care device.

In some embodiments, once trained, the communication between a UE and the cloud is limited to a list of ID's (e.g., a list of encoded data corresponding to certain indicators of terms in the vocabulary) of the selected voice/formatted text prompts fed and feeding the neural networks on both ends of the system 300 (e.g., at the UE 204 and the other entity 206 in the system 200).

The example implementation of the training system 300 is described below, with reference to the apparatus 100 and system 200. In this example, the system 300 trains the NLP model in the cloud and then transfers this model to the UE 204 although in other examples, the system 300 may be implemented on a local machine. The system 300 comprises certain blocks that may be implemented in the cloud, for example, with expert input (e.g., a service provider with knowledge of a certain type of personal care device and/or the personal care regime associated with this type of device). Certain blocks may be omitted when implementing the training system 300.

Block 302 of the system 300 refers to a database comprising certain data such as historical data about a plurality of users (which may include data on the present user) of the certain type of personal care device. This data may refer to the users' interaction with a personal care device, for example, while carrying out the personal care regime. Such data about the users' interaction may include contextual information indicative of the user experience. The database (or a different database accessible to the system 300) may further comprise input (e.g., a protocol, vocabulary list associated with the personal care regime, etc.) from an expert such as an expert on the personal care device (e.g., a professional with knowledge or a skill relevant to the personal care regime). Certain patterns may be noted in the data, which can be leveraged to formulate a protocol specifying how to react to certain user input (e.g., the user says their skin is irritated and then the NLP model understands contextual information from the user input in order to recommend a course of action for the user based on the protocol). In some cases, the protocol may be formulated using expert knowledge about the personal care device and/or personal care regime, for example, with or without input from the historical data from the plurality of users. In some cases, the database may comprise updated information, for example, derived from the user of the UE 204. In some cases, the historical data may comprise natural language representation of the user interaction such as user data samples comprising audio data and/or formatted text.

At block 304, the data from block 302 is processed with available (e.g., pre-existing or additional human) annotation to create a vocabulary associated with the type of personal care device. This vocabulary is stored in a vocabulary database 306 (e.g., in the cloud). In some cases, the data from block 302 may include sensor measurement(s) and/or contextual information for training purposes. The sensor measurement(s) and/or contextual information may be used to 'annotate' certain data. For example, certain combinations of sensor measurement(s) and/or contextual information may be indicative of a certain user behavior or experience, which may improve the accuracy of the training. Further, certain preconditions may be associated the sensor measurement(s) and/or contextual information, which may also be indicative of a certain user behavior or experience.

At block 308, the data from block 302 is processed to train a dialog protocol (e.g., comprising appropriate responses to user input). This training may use available (e.g., pre-existing or additional human) annotation to create a dialog-intelligent protocol, which may map events (e.g., data about what has happened during a personal care regime), actions (e.g., user behavior during use of the personal care device) and vocabulary used by the users (e.g., as stored in the vocabulary database 306).

As a result of the training at block 308, an NLP model 310 may be created. This NLP model 310 may be tested, at block 312, using control data 314 from another data set comprising data about a plurality of users' interactions with a personal care device to determine if the NLP model 310 is working with sufficient prediction accuracy. For example, the testing may involve handling and evaluating certain events from control data 314 based on existing knowledge (e.g., expert knowledge about protocols), to determine if the outcome of the NLP model 310 corresponds to the expected outcome. In other words, the NLP model 310 may be tested to determine if the correct protocol, as determined by existing knowledge and/or expert input, is suggested by the model 310. This may involve suggesting certain dialog in response to certain user input where that input is associated with certain contextual information. In some cases, sensor measurement(s) may form part of the input providing contextual information. In response to determining that the NLP model 310 could be improved, feedback may be sent to the vocabulary database 306 to improve the model training at block 308.

Once the NLP model 310 has been sufficiently trained, it may be transferred at block 316 to a user's UE 318 (e.g., UE 204). In addition, the vocabulary from the database 306 may be transferred to the UE 318.

The UE 318 may then implement the NLP model 310 as the user device 318 collects user data. The UE 318 may be trained used the user's data samples to personalize its response to the user. Where a term or a context of the term is not understood according to the NLP model 310, the user data sample comprising the term may be sent to the cloud for further analysis, which may lead to the NLP model 310 being updated before an update is returned to the UE 318 to update the NLP model 310 on the device. In some cases, the encoded data generated/recognized by the UE 318 may transfer data to/from the cloud in order to facilitate the training process and/or to allow a service provider implemented at the cloud to collect user data, e.g., for service improvements.

Fig. 4 shows another apparatus 400 for communicating data according to various embodiments. The apparatus 400 may improve data communication in a similar manner to the apparatus 100. In this embodiment, the apparatus 400 comprises the processing circuitry 102 of the apparatus 100 and may implement certain modules to support the functionality of the apparatus 100/400. Reference is made to the previous figures, where appropriate. Where appropriate, certain modules described below may be omitted from the apparatus 400.

The apparatus 400 comprises processing circuitry 402 for implementing the modules described below.

In some embodiments, the apparatus 400 comprises a memory 404 storing a mapping between at least one term of the vocabulary and corresponding encoded data representative of the at least one term. In use of the apparatus 400, in response to recognizing the term in the user data sample, the generating module 106 (implemented by processing circuitry 102) is configured to access the memory 404 to generate the encoded data corresponding to the recognized term.

In some embodiments, the recognition module 106 is configured to interpret the recognized term from the vocabulary associated with the personal care regime and/or a sensor measurement associated with a user's personal care regime based on a protocol stored in the memory 404. The protocol may specify a reaction to the interpreted term and/or sensor measurement. The reaction may comprise natural language, such as generated by a dialog synthesizer. An input to the synthesizer may be interpreted by a trained AI model to construct the natural language-based reaction. The apparatus 400 is configured to cause a user interface (e.g., of the personal care device or another UE) to present the reaction to the user. As already mentioned, a sensor measurement may be obtained, which may be used to determine a reaction based on a protocol stored in the memory 404. For example, the sensor measurement may indicate too much pressure and the apparatus 400 may indicate a reaction comprising a natural language representation indicating to the user to reduce pressure. This reaction may be presented in an audio data format via a user interface such as a speaker and/or visually via a user interface such as a display.

In some embodiments, the processing circuitry 402 of the apparatus 400 comprises a receiving module 406 configured to receive, from another entity (such as another UE or the cloud), information for implementing functionality of the recognition module 104 and/or generating module 106. For example, the receiving module 406 may interpret data received from the other entity and use this data to implement and/or update the functionality of the apparatus 100/400. Such information may comprise, for example, model information to update the NLP model of the apparatus 100/400.

In some embodiments, information is received in response to a request sent by the apparatus 400 to the other entity. The request may comprise an indication of unrecognizable information in the user data sample and/or a sensor measurement (e.g., the protocol does not provide an appropriate response, the context of the user data sample and/or sensor measurement is not understood by the apparatus 400 and/or the user data sample is not understood for some other reason). The information received in response to the request may comprise: an update for the NLP model; at least one term, recognized by the other entity, in the user data sample; and/or contextual information associated with the at least one term recognized by the other entity and/or the sensor measurement.

In some embodiments, the transmitting module 108 (implemented by the processing circuitry 102) is configured to transmit the request in response to the recognition module 104 being unable to recognize at least part of the user data sample and/or interpret the sensor measurement.

In some embodiments, the received information comprises a response statement generated (e.g., by the other entity) in response to the term indicated by the encoded data transmitted by the transmitting module 108. The response statement may be in the same data format as the encoded data. For this purpose, the processing circuitry 402 of the apparatus 400 further comprises a conversion module 408 configured to convert the response statement to a corresponding natural language representation of the term based on a mapping between the response statement and construction information for constructing the natural language representation of the term. For example, where the response statement is to be presented by an audio user interface such as a speaker, the construction information may comprise audio synthesizing information for synthesizing audio data corresponding to the response statement. By retaining the construction information on the apparatus 400, there may be no need to communicate excess or any information via the communication channel in order for the user to receive the response statement in the natural language representation.

In some embodiments, the processing circuitry 402 comprises a dialog management module 410 configured to receive an indication of the recognized term and/or contextual information associated with a user experience. The dialog management module 410 is further configured to generate a response to the indication based on a protocol. The response may comprise information for updating the NLP model and/or a request for further information to be collected from the user in accordance with the protocol.

The dialog management module 410 may implement a dialog feedback system, which may be used to train the dialog management module 410 to react in a personalized way to user input. For example, the dialog management module 410 may ask a series of questions of the user and, in response to the user feedback received in response to each question, the dialog management module 410 may become trained. By way of example, the dialog management module 410 may train or update a protocol (which may initially be expert-determined) in accordance with the user feedback so that the protocol can become better adapted to understand the user (e.g., the protocol can become personalized to the user such that it can be considered to be a 'user protocol', as described below). Thus, once trained, the dialog management module 410 may be better at understanding the user and may be able to recommend an appropriate course of action to take to improve the user experience. An example technique for improving the understanding may involve the 'five whys' technique. An expert-defined protocol may implement a similar technique and may be trained by the dialog management module 410 using the user feedback in order to better or more quickly understand the user (e.g., once trained, the protocol may return the most relevant reaction to assist the user based on their input).

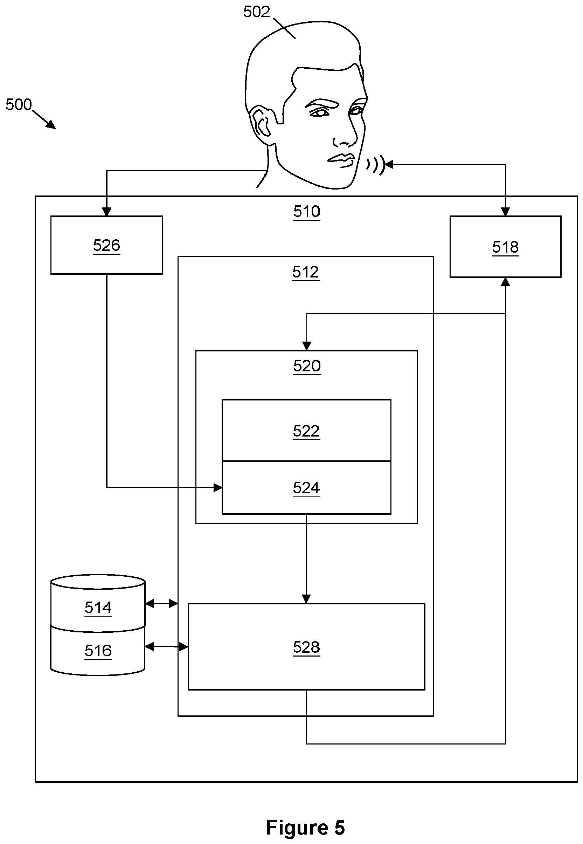

Fig. 5 shows a system 500 for implementing certain embodiments described herein. The system 500 comprises certain modules which may have the same or similar functionality to certain modules described in relation to the apparatus 100, 400 and systems 200, 300. In some embodiments, certain modules may be combined, omitted or otherwise modified from the structure presented in Fig. 5. Alternative implementations may involve certain modules being implemented by another device. For example, where different UEs are present (e.g., a personal care device, an IoT device and/or an edge device), the functionality implemented by the system 500 may be shared across these different UEs (e.g., one of the UEs implement the functionality of the apparatus 100, another UE may take a sensor measurement, another UE may record audio, etc).

This system 500 refers to an interaction between a user 502 and a UE 510 such as a personal care device (although it could be another UE such as a mobile phone or IoT device). The UE 510 comprises processing circuitry 512 for implementing the functionality of certain apparatus, methods and/or tangible machine-readable media described herein. The UE 510 comprises a memory 514 communicatively coupled to the processing circuitry 512. The memory 514 may store any appropriate information (e.g., NLP model information, user data samples, protocols (e.g., a user protocol such as a pre-determined protocol trained based on user input), historic information/data obtained from the user as part of the previous dialog and/or sensor measurements, etc) for implementing the functionality of the UE 510, as described below. In some cases, the memory 514 comprises a database 516, which may be accessible to certain modules of the UE 510 although in other cases such modules may comprise the database 516 itself.

In this system 500, the UE 510 further comprises a user interface 518 (e.g., a microphone, display screen, etc) for obtaining an input such as a user data sample (e.g., audio data and/or formatted text) from the user 502. In some cases, the user interface 518 may also provide an output such as audio (e.g., via a speaker) and/or visual information (e.g., via a display screen). In some systems, the user interface 518 may be implemented by another UE (e.g., a user phone, not shown) communicatively coupled to the UE 510 via a communication channel for transmitting encoded data and/or another data type such as raw audio data between the UEs.

The processing circuitry 512 comprises a recognition module 520, which may implement the same or similar functionality to the recognition module 104 of Fig. 1. The recognition module 520 may comprise a natural language recognition module 522 and/or a context recognition module 524. The natural language recognition module 522 may recognize, in a user data sample, a term from a vocabulary associated with a personal care regime. The vocabulary may be stored in the memory. Where present, the context recognition module 524 may be able to determine contextual information associated with the term. Such contextual information may be determined from a protocol (e.g., stored in the memory 514) such as a pre-determined protocol or a user protocol indicating possible reactions to user input and/or determined based on previous user input obtained from the user interface 518.

The UE 510 further comprises a sensor 526 for obtaining sensor measurements, although a different UE communicatively coupled to the UE 510 may comprise such a sensor 526. The sensor 526 may feed sensor measurements to the context recognition module 524. Such sensor measurements may assist with understanding the context. For example, a sensor measurement may be associated with user dialog not recognized or partially recognized by the recognition module 520. Thus, the sensor measurement may assist with recognizing the user dialog, and vice versa. An example implementation may be the user indicating they have pain (e.g., natural language such as 'ouch') but the recognition module 520 does not understand the context (e.g., if it has not been personalized/trained with the characteristics of the user's speech). A sensor measurement may be obtained (prior to, during or after the user dialog) indicating that too much pressure is applied between the user's skin and the personal care device. Thus, the context recognition module 524 may associate these events together.

The processing circuitry 512 further comprises a dialog management module 528, which may implement the same or similar functionality to the dialog management module 410 of Fig. 4. The output from the recognition module 520 may be fed to the dialog management module 528, which may use a protocol (e.g., a user protocol) and/or previously-obtained user data to determine or update a reaction to the output received from the recognition module 520. The reaction may be fed back to the user interface 518 as part of a user-dialog feedback system. Further potential functionality of the dialog management module 528 is described in more detail below.

Although the UE 510 comprises the recognition module 524, which may have similar functionality to the recognition module 104 of the apparatus 100, other modules of the apparatus 100 (i.e., the generating module 106 and the transmitting module 108) are not depicted in Fig. 5. In some cases, the UE 510 comprises processing circuitry implementing the functionality of one or both of these modules 106, 108. Although not shown, the UE 510 may interact with another UE or the cloud, for example, when communicating data as described herein. Other embodiments described herein which refer to improving the user experience may or may not make use of modules involved in the data communication although they may make use of certain functionality provided by the recognition module 524 and/or the dialog management module 528.

Certain functionality of the system 500 may be implemented by other apparatus, methods and machine-readable media described herein, such as those described in relation to the section below regarding user-device interaction.

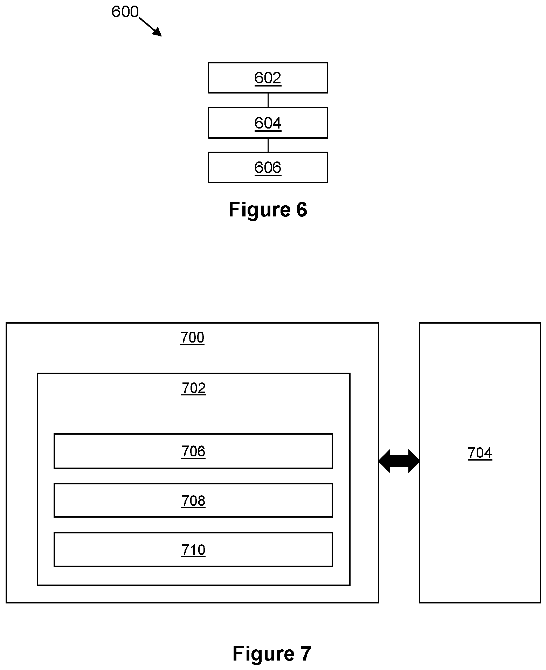

Fig. 6 shows a method 600 (e.g., a computer-implemented method) for communicating data. The method 600 implements the same functionality as the apparatus 100. Thus, each block of the method 600 corresponds in functionality to the modules 104 to 108 of the apparatus 100. The method 600 may be implemented by a UE such as a personal care device, base station, etc. Further methods may be specified with functionality corresponding to that provided by certain apparatus described herein (e.g., the apparatus 100, 400).

The method 600 comprises, at block 602, recognizing, using a natural language processing, NLP, model, a term from a vocabulary associated with a personal care regime in a user data sample comprising a natural language representation of the term.

The method 600 comprises, at block 604, generating encoded data indicative of the term, wherein the encoded data uses less memory than the user data sample.

The method 600 further comprises, at block 606, transmitting the encoded data.

Fig. 7 shows a tangible machine-readable medium 700 storing instructions 702 which, when executed by at least one processor 704, cause the at least one processor 704 to implement certain methods described herein, such as the method 600, or implement the functionality of the apparatus 100,400.

In this embodiment, the instructions 702 comprise instructions 706 to implement block 602 of the method 600. The instructions 702 further comprise instructions 708 to implement block 604 of the method 600. The instructions 702 further comprise instructions 710 to implement block 606 of the method 600. In some embodiments, the instructions 702 comprise additional instructions to implement the functionality of certain embodiments relating the methods or apparatus described herein.

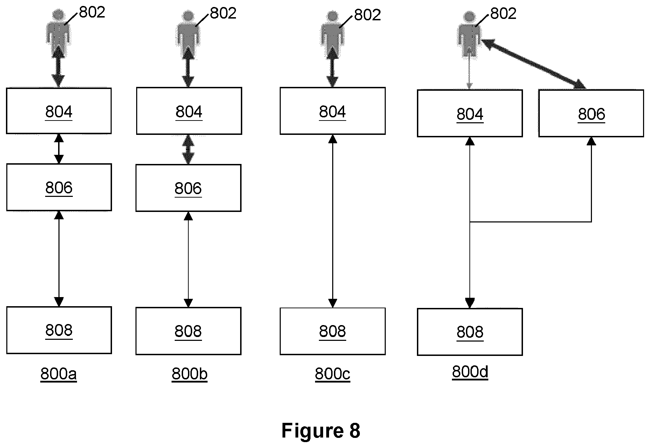

Fig. 8 shows some different scenarios to which certain apparatus, methods and machine-readable media described herein may be applied according to certain embodiments. Fig. 8 shows four different systems 800a, 800b, 800c, 800d representative of these scenarios. In this regard, reference is made to the system 200 of Fig. 2. Each system 800a-d refers to a communication arrangement involving at least one UE and the cloud where at least one communication channel provides a low data-rate communication between at least two entities in the system 800a-d. In use of these systems 800a-d, data may be communicated between the UE and the cloud to train/use an NLP model, facilitate communication of data with minimal resource usage and/or implement an improved user experience.

System 800a refers to a scenario with two UEs and the cloud. In use, a user 802 interacts with a first UE 804, which may collect a user data sample (e.g., dialog/audio) from the user 802 and/or a sensor measurement. The first UE 804 may comprise, for example, a personal care device or another IoT device which is hand-held or wearable. The first UE 804 implements the functionality of certain apparatus, methods and/or machine-readable media described herein (e.g., at least one of the apparatus 100, 400, method 600 and/or machine-readable medium 700). In this embodiment, the first UE 804 can train the NLP model and/or recognize contextual information based on the user data sample and/or sensor measurement.

The first UE 804 is communicatively coupled to a second UE 806 via a narrowband communication channel (or any communication channel with limited resource for communicating data rapidly). The second UE 806 may comprise a base station, mobile phone or other UE. The second UE 806 may act as a relay between the first UE 804 and the cloud 808. The second UE 806 implements the functionality of certain apparatus, methods and/or machine-readable media described herein (e.g., at least one of the apparatus 100, 400, method 600 and/or machine-readable media 700). In this embodiment, the second UE 804 can train the NLP model and/or recognize contextual information based on the user data sample and/or sensor measurement.

Either one of or both of the first and second UEs 806 may be involved in training or using the NLP model.

The cloud 808 may also implement the NLP model. As described previously, in some implementations, the NLP model may be initially trained by one entity (e.g., in the cloud) and then certain model information may be transferred to another entity (e.g., the first and/or second UE 804, 806) to enable certain functionality described herein such as recognition of terms from the vocabulary and/or interpreting the context of the user data sample and/or sensor measurement. Where certain information is not understood, an indication of the user data sample and/or sensor measurement may be transferred to the second UE 806 or the cloud 808 for further processing, and then model information and/or an updated database entry may be received by the first UE 804 to facilitate better understanding of the certain information. Thus, over time, the cloud 808 may be used less and less for training since the UE 804, 806 may become better trained through self-training (e.g., using user input and/or sensor measurements to update the functionality of any modules that use AI) and/or through the cloud-based training described below.

In this embodiment, the communication channel between each of the first UE 804, second UE 806 and the cloud 808 is implemented by a narrowband communication channel (or any communication channel with limited resource for communicating data rapidly). Thus, the functionality provided by the apparatus 100, 400 and associated methods and machine-readable media may be implemented by each of these entities (e.g., the first UE 804, second UE 806 and the cloud 808) of the system 800a to transmit encoded data. In other similar words, each of these entities may implement certain functionality of the NLP model for various purposes, including training or using the NLP model, communicating information about the NLP model and/or improving the user experience.

The scenario depicted by system 800a may refer to various situations. For example, in one situation, the first and second UEs 804, 806 communicate with each other with a short-range communication protocol (e.g., to save power). In such a situation, the second UE 806 may communicate with the cloud using some longer-range communication technique, e.g., a wired connection or a cellular technology such as 3G, 4G, 5G, etc. Even though such cellular technologies may support a high data communication rate, certain embodiments described herein may reduce the amount of data sent via such communication channels, which may reduce cost and/or resource usage. In one situation, the second UE 806 may relay data. In another situation, the second UE 906 may support implementation of the NLP model (e.g., for training or other use). In another situation, the second UE 806 may assist the first UE 804 with processing of the NLP model, and vice versa, for example to decrease processing time and/or reduce the processing resource needed in each device 804, 806.

System 800b refers to a similar scenario to that of system 800a. However, the first UE 804 does not implement any functionality regarding the NLP model. Rather, this functionality is implemented by the second UE 806. An example situation associated with the system 800b may refer to where the first UE 804 merely collects the user data sample and/or a sensor measurement and transfers this data to the second UE 806 (e.g., without encoding the data as defined by certain embodiments described herein). The second UE 806 and cloud 808 may then communicate in accordance with certain embodiments described herein (e.g., via a narrowband communication channel).

System 800c refers to a slightly different scenario to those represented by systems 800a, 800b. In this embodiment, there is a first UE 802 and the cloud 808 with direct communication therebetween, for example, via low bit-rate communication channel.

System 800d refers to a similar scenario to system 800a but with the first UE 804 and second UE 806 communicating with the cloud 808 using separate communication channels. In this embodiment, the first UE 804 takes a sensor measurement while the second UE 806 takes a user data sample (e.g., user audio) although in some cases both UEs 804, 806 may obtain such data. An example implementation of system 800d could refer to an audio-enabled personal care device (e.g., a first UE 804) and a sensor-equipped phone or other edge device (e.g., a second UE 806), or vice versa.

The systems 800a-d refer to various possible implementations where at least one communication channel is narrowband (or at least has limited communication resource available due to network restrictions and/or cost). Different devices at the edge may be assigned different tasks including: user data sample collection, sensor measurement, training/personalizing the NLP model, interacting with the cloud 808 to initialize the NLP model and/or update the NLP model and/or using the NLP model to facilitate an improved user experience, etc.

### User Experience

Certain embodiments described above refer to communicating data to reduce the amount of data transmitted by implementing an NLP model. Certain embodiments described above also refer to using the NLP model as part of a dialog feedback system involving a personal care device. Certain embodiments described above refer to training the NLP model at a UE or in the cloud, for example, by using information from a protocol (e.g., based on information obtained from a plurality of users and/or expert input) for initial training and potentially using additional information obtained from a user to further train/personalize the NLP model (e.g., to generate a 'user protocol'). Such embodiments may reduce resource (e.g., hardware, software, communication channel resources, etc) usage when communicating or storing the data collected or generated in accordance with certain embodiments described herein. In addition, the reduced resource usage may help to provide an enhanced user experience. Further apparatus, methods and machine-readable media relating to improving the user experience are described below.

The interaction between the user and a UE such as a personal care device may influence the user experience. Such a user experience may be enhanced by making use of various sources of information. For example, sensors may take various measurements while a user is carrying out a personal care regime. This measurement data may be used to provide user feedback. In addition to sensor measurements, a user may input data such as in natural language form (e.g., user dialog), which may be used to provide feedback for the user. Certain apparatus, methods and machine-readable media described herein may leverage sensor measurement data and/or user input in order to improve the user experience by providing a more relevant reaction to user input and/or a quicker reaction to the user input. apparatus, methods and machine-readable media described above and apparatus, methods and machine-readable media described below may relate to concepts which support each other to, for example, improve the user experience and/or efficiently use available resources. In other words, certain features of apparatus, methods and machine-readable media described above may be combined with certain features of apparatus, methods and machine-readable media described below in order to provide such improved user experience and/or efficient resource usage.

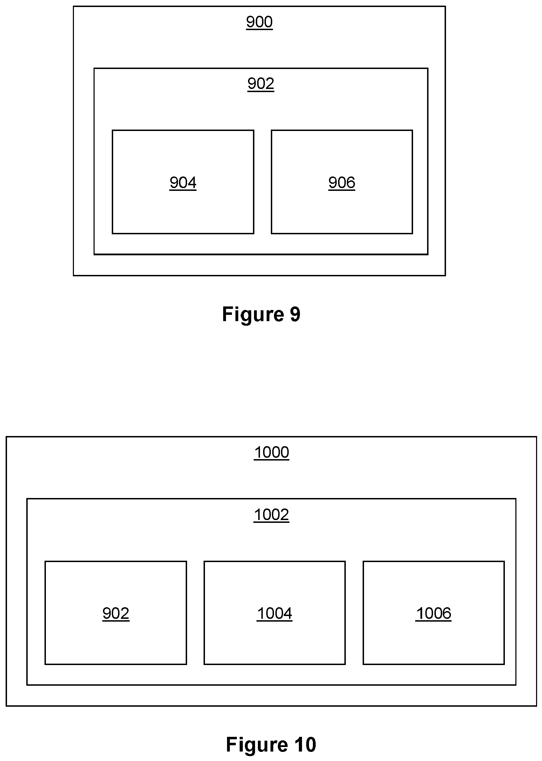

Fig. 9 shows an apparatus 900 for improving user experience. The apparatus 900 may improve the user experience by making use of contextual information in user dialog to better understand the user input. Certain modules of the apparatus 900 may implement the same or similar functionality to certain modules described above. For example, the recognition module 104 of Fig. 1 may be configured to train the NLP model based on user data.

The apparatus 900 comprises processing circuitry 902 for implementing the modules below. In one implementation, the apparatus 900 may be implemented by at least one processor (e.g., corresponding to the processing circuitry 902) for executing instructions stored in a machine-readable medium, where those instructions are configured to implement the functionality described below. In another implementation, the apparatus 900 may be implemented by at least one dedicated processor such as an Artificial Intelligence (AI) engine.

The apparatus 900 may be implemented by a UE such as a personal care device, base station or other UE or the apparatus 900 (or its corresponding functionality via a method or machine-readable medium) may be implemented in the cloud.

The processing circuitry 902 comprises a determining module 904 and a dialog management module 906.

The determining module 904 is configured to interpret natural language in a user data sample associated with use of a personal care device as part of a personal care regime to determine contextual information indicative of a user interaction with the personal care device.

The dialog management module 906 is configured to use the contextual information to determine a reaction to the user data sample in accordance with a user protocol stored in a memory accessible to the apparatus. The user protocol may be considered to be a user-personalized protocol, which may be trained or personalized based on user input (e.g., user dialog and/or sensor measurements), expert input and/or historical data.

The apparatus 900 may have similar functionality to at least part of the apparatus 400 of Fig. 4 and/or the UE 510 of Fig. 5. For example, certain functionality of the recognition module 520 of Fig. 5 may be implemented by the determining module 904. In addition, certain functionality of the dialog management module 410 of Fig. 4 and/or dialog management module 528 of Fig. 5 may be similar to the functionality of the dialog management module 906. In Fig. 4, a dialog feedback system is implemented, which may be used to train the NLP model to react in a personalized way to user input and/or train the system to have an NLP model (i.e., the original model may not exhibit NLP capabilities but it may be trained, using the dialog, to extend the original model to comprise an NLP model).

The apparatus 900 of Fig. 9 may implement certain functionality of the dialog management module 410 (e.g., as part of the dialog feedback system). In this case, the apparatus 900 does not implement certain other features of the apparatus 400 (e.g., the encoding data and/or transmitting data part implemented by the apparatus 100) although in some cases such other features may be implemented by the apparatus 900.

Through use of the apparatus 900, the user may receive feedback on their personal care regime and usage of the personal care device. Such feedback may help to reinforce and/or personalize the functionality of the apparatus 900, through additional training, such that the user feedback may be improved. By gaining an understanding the contextual information in the user data, the apparatus 900 may provide an improved user experience, for example, due to a more relevant or faster response to user queries, feedback and/or sensor measurements. By way of example, without training a user dialog system may provide a relevant or useful user interaction 70% of the time. By performing the training, using the user dialog and/or sensor measurements, the relevancy or usefulness of feedback may increase by up to the remaining 30%.

A system that implements only a sensor-based algorithm to determine how a user is interacting with a personal care device may have a relatively restricted understanding of the user experience. By combining such information with a user-dialog feedback system, which improves contextual understanding, may increase understanding, for example, 99% of the time. Such a feedback system with the contextual understanding may replicate dialog with a personal health expert in order to provide a 'diagnosis' (e.g., help or a recommendation) based on the context (i.e., sensor readings, pre-event information collected prior to the dialog, the voice (e.g., tone) and choice of words, etc). The dialog feedback system may 'diagnose' using conversation with the user, such as using the 'five whys' technique to refine understanding of the user (e.g., a first user answer may not be understood but a subsequent answer may be better understood, which may place the first user answer in context, and thereby train the system). In this manner, the system is personalized through training/learning so that in subsequent use, the system may better (and more quickly) understand the user (e.g., by asking fewer questions and/or providing a relevant reaction to the user input more quickly). The level of difference achieved by system, based on dialog feedback to understand the context, may be to raise performance (e.g., prediction accuracy) from 70% to, for example, 99%. The combination of sensor readings and dialog feedback may improve performance further. Thus, the dialog may train existing models to better reflect the user's speech style, language, etc. while also providing an improved user experience since they may obtain more accurate and faster feedback results.

The model(s) used to implement the functionality of the apparatus 900 may be trained using the user dialog and/or sensor measurements. A pre-determined protocol specified based on a plurality of users' data and/or expert input may be modified as a result of the training. For example, the pre-determined protocol may associate certain user input with a certain reaction after going through a series of questions (e.g., the 'five whys' technique). For example, the apparatus 900 may not be able to understand the contextual information in the initial user input (e.g., the term used in the dialog is not associated with any terms mentioned in the protocol). However, by performing the training (e.g., based on annotation and/or expert input on the user input and feedback to the dialog generated by the model based on the protocol), the apparatus 900 may gain a better understanding of the initial user input if it is associated with another part of the protocol. Accordingly, the training process may generate an improved model and/or user protocol, which can be used for personalized feedback for the user.

It has been mentioned already that sensor measurements may be used. Thus, the determining module 904 may be configured to determine contextual information indicative of the user interaction with the personal care device based on a sensor measurement associated with use of the personal care device as part of the personal care regime. The system 500 depicts an example of a sensor 526 which may be used to provide the sensor measurement. Such a sensor 526 may be part of any UE, for example, the personal care device, a mobile phone or an IoT device, etc. Examples of sensors include: a camera, microphone, pressure sensor, temperature sensor, motion sensor, water sensor and any other type of sensor which might obtain information which might provide data for improving the user experience in connection with the personal care regime. Although a sensor 526 may provide the information for the determining module 904 (e.g., for 'live' or current measurements), sensor measurement data (and indeed other user input such as user dialog) may be stored in the memory 514. In this manner, historical data from the user (collected prior to the current interaction with the user) may be accessed and used for determining the context.

The sensor measurement may be used in a similar way to the additional feedback received from the user based on the user dialog. For example, the sensor measurement may be associated with user dialog (e.g., due to the timing of when the user dialog and sensor measurement) and/or may be associated with a certain part of the protocol. An example scenario may refer to a pressure sensor indicating that too much pressure is being applied on the skin by the personal care device and therefore causing discomfort. Such a pressure reading may directly indicate, via the protocol, that too much pressure is being applied. However, in some cases, different users may have different experiences which means that the (e.g., pressure) threshold at which discomfort is experienced depends on the user. Thus, by understanding contextual information in the user dialog in combination with the sensor measurement, it may be possible to provide improved user feedback by associating the user dialog with the sensor measurement when performing the training process. For example, the protocol may be updated in accordance with a user experience, which may depend on their dialog with the dialog management module 906 and/or sensor measurements obtained.

Other scenarios with the various sensors may include: a camera detecting skin irritation after shaving, a microphone picking up non-verbal communication (e.g., sounds made when the user is carrying out the personal care regime), a motion sensor to detect movement indicative of various information such as a less efficient shaving motion, potential damage due to dropping the shaver, etc. Although these examples mention shaving, other types of personal care devices such as oral healthcare devices may feature sensors for obtaining measurements.

The dialog management module 906 may be configured to generate an indication of the reaction for causing a user interface (e.g., the user interface 518 of Fig. 5) to interact with a user of the personal care device. For example, the dialog management module 906 may provide the reaction based on the protocol and cause an appropriate part of the system (e.g., system 500) to respond accordingly.

The indication may comprise a message to be issued, via the user interface, to a user of the personal care device and/or a control instruction to control an operation of the personal care device. For example, the user interface may comprise a speaker for playing an audio representation of the message and/or a screen for displaying the message (e.g., via an icon, formatted text or other visual indication). The control instruction may change how a certain component of the personal care device operates to thereby improve user experience. For example, in response to determining that a shaver cutter speed is not appropriate for the user's experience, the control instruction may cause the personal care device to change the cutter speed setting to improve performance.

The determining module 904 may be configured to implement a natural language processing, NLP, model to interpret natural language in the user data sample associated with use of the personal care device as part of the personal care regime and determine the contextual information indicative of the user interaction with the personal care device. As mentioned previously, the NLP model may or may not be AI-implemented.

The dialog management module 906 may be configured to implement an artificial intelligence, AI, model to determine the reaction to the user data sample in accordance with the user protocol stored in the memory accessible to the apparatus.

The AI model of the dialog management module 906 may be configured to personalize the user protocol for a user of the personal care device based on the user data sample.

The dialog management module 906 may be configured to use a previously-obtained protocol stored in the memory to determine a user query based on the user data sample. The dialog management module 906 may be configured to cause a user interface (e.g., a speaker and/or screen) to present the user query at a user interface. The AI model may be configured to personalize the user protocol based on an additional user data sample received in response to the user query. As mentioned previously, the dialog management module 906 may implement a dialog feedback system in which the user protocol is personalized based on the dialog exchange between the user and the dialog management module 906. The 'previously-obtained' protocol may refer to a previous update to the user protocol (following training or personalization) or a pre-determined protocol as described below.

The AI model may be initially trained based on a pre-determined protocol. The pre-determined protocol may be based on a user behavioral database comprising information about a plurality of users' interaction with a specified type of personal care device associated with the personal care regime and/or may be based on expert input about how users interact with the specified type of personal care device associated with the personal care regime.

The dialog management module 906 may be configured to update the user protocol using a dialog exchange between the dialog management module and a user of the personal care device based on the dialog management module 906 making an association between a previous version of the user protocol (e.g., the previously obtained protocol) and contextual information in dialog received from the user.

The dialog management module 906 may be configured to update the user protocol by associating a sensor measurement obtained in relation to use of the personal care device by making an association between a previous version of the user protocol and contextual information determined from the sensor measurement. Thus, the sensor measurement may be used to improve the user experience by becoming associated with part of the previous version of the user protocol, which may help to train the AI model to better understand subsequent user dialog.

As referred to previously, the dialog system and/or sensor measurements may help to train a model (e.g., NLP model and/or the model for providing user feedback based on dialog). Such a model may be built by causing the system to ask the user questions (e.g., 'annotating' the unknown/not understood data) in order to perform training. For example, a sensor reading may be considered strange or cannot be placed in context (in accordance with the current user protocol) or is simply not understood. In one implementation, the system may ask the user for feedback which might help the system understand what this means. In another implementation, the system may send a question to an expert (e.g., via the cloud) if the system cannot ascertain by itself, with user feedback, what is meant by the sensor reading. In an example implementation, a strange/undefined sensor reading in combination with a user response may not yield an answer because the sensor reading is technical in nature. Thus, an expert could provide feedback based on the sensor reading with user feedback 'annotating' the sensor reading event, which may be used to train the system to better understand the next event (e.g., a sensor reading), if it happens again or if a similar event happens again.

Fig. 10 shows an apparatus 1000 for improving user experience. The apparatus 1000 may improve the user experience by making use of contextual information in user dialog to better understand the user input. The apparatus 1000 may be implemented in a similar way to the apparatus 900. Further, the apparatus 1000 may provide the same or similar functionality to the apparatus 900 of Fig. 9. In this regard, the apparatus 1000 comprises processing circuitry 1002, which comprises the processing circuitry 902 of Fig. 9.

The processing circuitry 1002 comprises certain modules described below, which may facilitate or support certain functionality associated with the modules of the processing circuitry 902. The processing circuitry comprises a receiving module 1004 and a transmitting module 1006.

The receiving module 1004 is configured to receive, from another entity via a communication channel, model information for implementing functionality of the determining module 904 and/or dialog management module 906. The other entity may refer to another UE or the cloud. The communication channel may be any communication channel, such as one supporting the data communication described above or another type of data communication. The model information may refer to NLP model information and/or AI model information. The model information may comprise a protocol (e.g., the pre-determined protocol and/or an updated/trained user protocol).

In cases where the apparatus 1000 understands the user and the contextual information, the determining module 904 and/or dialog management module 906 may provide an expected user experience, e.g., with accurate reactions to the user input and/or providing feedback for the user that the user considers to be high quality. However, in cases where the apparatus 1000 is unable to understand the user and/or the contextual information, the user data sample and/or sensor measurement may be sent, as described below, to the other entity. The other entity may be a service provider (e.g., providing a service to support the user's personal care regime, for example, as part of a support package associated with the personal care device). This other entity may user their expertise (e.g., human experts), databases and/or AI engine(s) to train/update the model(s) to provide an appropriate reaction that may improve the user experience. Information about the improved model(s) (e.g., modified network weights in the case of a neural network) may be sent to the apparatus 1000 and used to update the user protocol and/or improve the functionality of the determining module 904 and/or the dialog management module 906. Further, the backend (e.g., operated by a service provider via the cloud) may have access to the various data/model updates by the various users, which may help with training and providing expert input to subsequent training queries received from user equipment which is struggling to understand the context/sensor measurements.

The transmitting module 1006 is configured to send, in response to the determining module 904 being unable to interpret at least part of the user data sample and/or the dialog management module 906 being unable to determine how to react to at least part of the user data sample, the user data sample for processing by the other entity to determine updated model information for updating the functionality of the determining module to interpret the user data sample and/or updating the functionality of the dialog management module to determine how to react to the user data sample.

Some example scenarios which make use of the apparatus 900, 1000 are described below.

In one example scenario where the user may apply too much pressure while shaving, a sensor measurement may register such pressure, which may be communicated with the cloud, which then provides some indication that too much pressure is being applied. However, the AI model may recognize that too much pressure is being applied and provide feedback for the user.

In another example scenario, the user may note that their skin feels irritated. Skin irritation may be caused by various issues, including too much pressure, blunt blades, low battery, wrong shaving direction, etc. However, it may not be possible to understand why the user has irritated skin without further data. However, the AI model may have access to various information (e.g., directly from the user via dialog) and may make a decision which leads to appropriate feedback for the user to improve their user experience.

By combining a sensor measurement with user dialog, the context of the sensor reading may be better understood (and vice versa) and thus the AI model may improve the user experience. A feedback loop may be formed or improved by the AI model to help provide advice for the user. If the sensor measurement is indicative of something strange happening, the AI model could cause a question to be asked of the user to find out what is happening and, in some cases, the conversation between the device and the user could be steered by the sensor readings. Further, the sensor measurement may be used to train the system so next time the system would understand the context and the event (e.g., the sensor measurement).

The user data sample and the sensor measurement may not be obtained at the same time. For example, the user data sample may be obtained and used to train the AI model. At another time, a sensor measurement may be obtained and used to further train the AI model. Even though the user data sample and the sensor measurement may be obtained at different times, they may be linked in some way, which may be learned by the AI model. For example, a sensor measurement may recognize that a certain amount of pressure is applied during shaving, but this may not be sufficient to cause an immediate concern. However, at another time, the user may remark that their skin is irritated (whether or not they are shaving at that time), and the dialog management module 906 may connect these two data points in order to train the AI model and next time be able to give the appropriate/correct feedback to the user. In use of the personal care device, another sensor measurement may recognize that the level of pressure is slightly too high and a user interface may be caused to indicate to the user, visually or audibly, that they may wish to reduce pressure because they previously indicated that their skin is irritated. Thus, the AI model may be trained to associate user data sample with a corresponding sensor measurement, even if those two data points are collected at different times. An example scenario may be that a shaver detects a strange sensor reading then, later in the day, when the user uses their mobile phone (e.g., another UE), an application running the phone may ask the user about the shaving. Thus, the sensor measurement may prompt some user dialog feedback mechanism, even if the data is acquired at different times. Further, the AI model may be personalized to the user, for example, if the pre-determine protocol may not necessarily indicate that certain user data is to be linked with a certain response to that user data. Data in this scenario may be used again to train the model, for example, to make the system smarter and enable better feedback and user experience.

The AI model may be initially trained using the information in the user behavioral database and the AI model is further trained by the dialog management module 906 based on the user data sample and/or the sensor measurement. Thus, as more data is collected, this may be fed to the dialog management module 906 to improve the model. In some cases, this information may be fed back to the service provider in order to further refine the pre-determined protocol.

The pre-determined protocol may comprise a plurality of potential reactions to input terms and input sensor measurements in the user behavioral database. The potential reactions and/or input terms may be listed in a 'vocabulary' that is based on the personal care device/regime (e.g., as referred to in relation to Fig. 1). The dialog management module 906 may be configured to associate the user data sample and/or the sensor measurement with at least one of the potential reactions based on the interpreted contextual information. For example, a potential reaction may refer to an instruction to inform the user that they need to change some element of their behavior (e.g., change style of shaving, etc) or implement some other change to improve the user experience (e.g., put the device on to charge, change a component that is worn out, recommend another product, etc). By interpreting contextual information (e.g., by use of the NLP model as referred to above), a user query or other statement indicative of there being some issue such as skin irritation, the dialog management module 906 may update the AI model to link the newly-obtained contextual information to at least one of the potential reactions. While using the personal care device, the AI model may be configured to provide feedback to the user based on the updated AI model.

The pre-determined protocol may be based on human expert input and/or a machine-learning model for analyzing historical information obtained from a plurality of users and the users' interaction with a specified type of personal care device associated with the personal care regime. As mentioned above, the interaction may depend on the specified type of personal care device. Thus, the pre-determined protocol may be tailored to the type of personal care device, which may reduce the size of the AI model and/or improve the prediction accuracy of the AI model.

In other similar words, the apparatus 900, 1000 may fuse certain sensor data (e.g., relating to motion, skin, temperature, etc) and conversational (e.g., audio) data related to a use case (e.g., the personal care regime). Use of AI technology may provide more and/or more reliable information compared to the individual sources of data. In other similar words, fusion of sensor measurement and user dialog may train the AI model to be capable of providing accurate prediction of an appropriate response to user input. In an example of shaving, a specified NLP questionnaire and/or user interaction in combination with motion sensor readings and motor monitor data may be used to inform or improve the user experience.

Thus, certain apparatus, methods and machine readable media described herein may improve user experience by measurement of the user experience in a user-friendly and cost effective way and may fulfil the (new) user expectations of higher performing connected personal care devices. Alone, sensors and corresponding algorithms may have certain limitations and may not provide feedback that matches the high level of user expectations. Adding more processing power, cameras and expensive sensing solutions may not be appropriate in certain consumer applications to stay cost effective. However, by training and distributing the AI model as described herein, certain apparatus, methods and machine readable media described herein may facilitate and improved user experience that meets or exceeds user expectations. In other similar words, a 'smart system' may be created or facilitated by certain apparatus, methods and machine readable media described herein. The system may behave in a manner appropriate for the context (e.g., through comprehension of the user dialog and/or sensor measurements) and for the user themselves (i.e., via the personalization of the system). The user may have a high confidence in the accuracy of the system through the relevant and personalized responses generated by the system. The user dialog may decrease the need for additional sensors (e.g., whereas lots of sensors and corresponding processing may be needed in some settings), the user dialog may improve the contextual understanding of the sensor measurement, which may decrease the need for needing to acquire potentially harder-to-interpret multiple sensor measurements. This system implementation may be closer to, or in some cases better than, a personal health expert providing help via dialog with the user.

Motion, motor and/or temperature sensors may help with providing user feedback and improve user experience to a certain level. Audio and conversational information may provide additional information such as breathing, heartrate, speed, accuracy, skin irritation metrics, oral healthcare metrics, etc. The feedback mechanism may implement a conversation-led (or diagnosis-like) approach to providing answers to user queries as part of a feedback mechanism. A sensor reading may be triggered based on user feedback. Some sensors may use a lot of power and it may not be desirable to use these sensors all the time. In this case, such sensors may be used when needed and/or based on an indirect request from the user. Thus, certain apparatus, methods and machine readable media described herein may improve feedback sensing, user experience and/or otherwise contribute to an overall connected experience. Audio and conversation can help with the user experience. The user's personal mood, experience and/or health may also be reflected in the audio, and thus the dialog in certain AI model-informed conversation may be directed by the sensor information collected while and prior to the conversation. Detecting data such as the heart rate and breathing in audio data can be improved using motion sensor data from the personal care device and/or may involve filtering out motion generated noise, tuning in on specific frequencies or events in the audio data based on motion information. Thus, the information present in multiple source may improve the reliability and accuracy of the feedback.

By combining data of the different sources to train the AI model with certain aims as described above, an improved version of the AI model may be deployed to certain UEs to perform real-time processing of data, which may be used to alert or inform users about certain feedback and/or or trigger an event via a user connected application (e.g., acquiring more information, changing a setting on the personal care device such as tuning a motor control protocol of a shaver to prevent overheating or changing the cutting depth of the saver to a safer, more suitable level personalized for the user, etc).

A trained AI model may be used to assist or implement a 'call bot' to handle user questions, social calls and assist in monitoring of the user's experience of the personal care device to thereby improve the user experience.

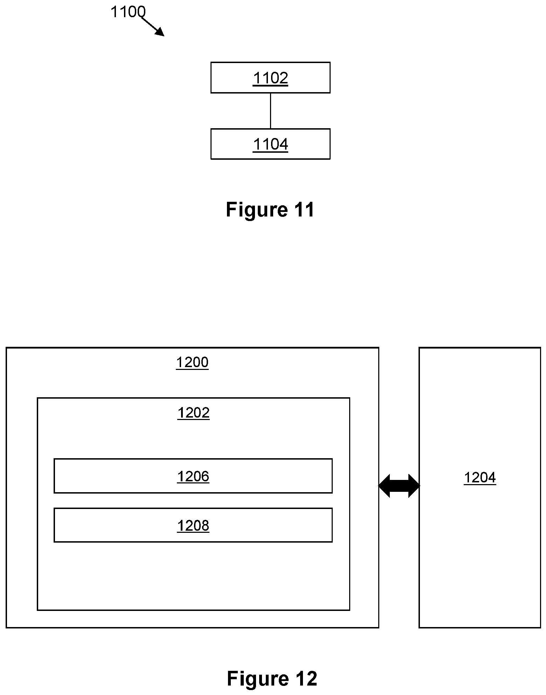

Fig. 11 shows a method 1100 (e.g., a computer-implemented method) for improving user experience. The method 1100 implements the same functionality as the apparatus 900. Thus, each block of the method 1100 corresponds in functionality to the modules 904 to 906 of the apparatus 1000. The method 1100 may be implemented by a UE such as a personal care device, base station, etc or by another entity such as the cloud.

The method 1100 comprises, at block 1102, interpreting natural language in a user data sample associated with use of a personal care device as part of a personal care regime to determine contextual information indicative of a user interaction with the personal care device.

The method 1100 further comprises, at block 1104, using the contextual information to determine a reaction to the user data sample in accordance with a user protocol.

Fig. 12 shows a tangible machine-readable medium 1200 storing instructions 1202 which, when executed by at least one processor 1204, cause the at least one processor to implement certain methods described herein, such as the method 900, or implement the functionality of the apparatus 900, 1000.

The instructions 1202 comprise instructions 1206 to implement block 1102 of the method 1100. The instructions 1202 further comprise instructions 1208 to implement block 1104 of the method 1100.

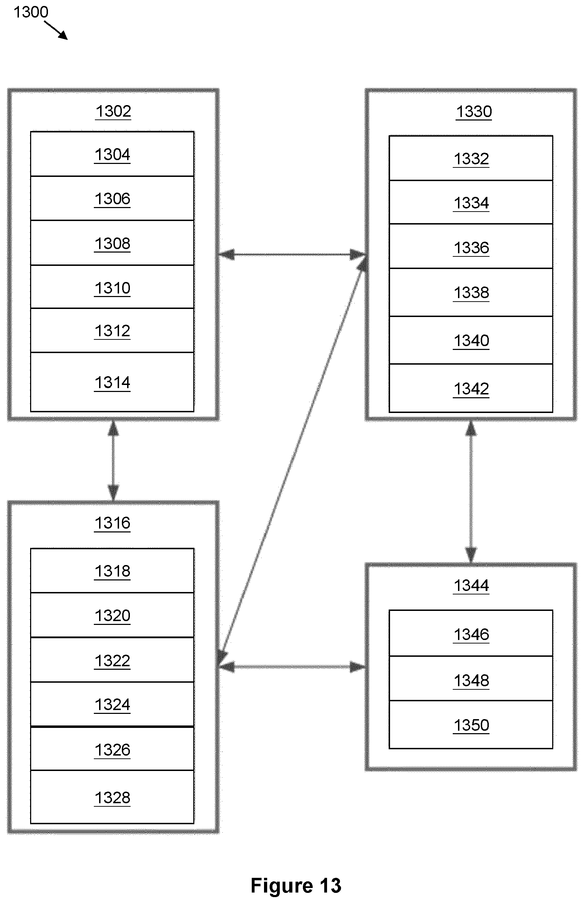

Fig. 13 depicts a system 1300 comprising the various entities for implementing certain apparatus, methods and machine readable media described herein. Certain devices may be omitted in certain settings. For example, system 200 of Fig. 2 shows two entities (e.g., a UE and the cloud). In this case, there are three UEs and the cloud involved in implementing the system 1300. Each entity comprises certain modules implementing certain functionality. However, not all modules may be present or used in some implementations, or a certain combination of these modules may be implemented to provide the improved user experience. Thus, the modules described below are exemplary and may not necessary be deployed or present for all applications of the system 1300.

The modules may be implemented by processing circuitry, hardware and/or software implementations. Thus, the modules may refer to different layers of the system 1300 architecture (e.g., from the application layer down to the physical layer).

A first UE 1302 such as a personal care device may comprise at least one of the following modules depicted by Fig. 13: a motion sensor 1304, optical sensor 1306, motor drive sensor 1308 (e.g., for a cutter), force/pressure sensor 1310, audio input 1312 (e.g., microphone) and/or an AI model engine 1314 (e.g., corresponding to functionality provided by certain apparatus such as apparatus 900, 1000). The first UE 1302 may comprise the sensors and locally-trained AI model. The audio input 1312 may be included because of the optimal user distance and additional sensing features provided by the associated sensors.

A second UE 1316 such as a base station or home 'hub' may comprise at least one of the following modules depicted by Fig. 13: audio input 1318, optical sensor 1320, audio output 1322 (e.g., a speaker), an AI model engine 1324 (e.g., corresponding to the functionality of certain apparatus such as apparatus 900, 1000), an NLP model engine 1326 for providing a user dialog system (e.g., corresponding to the functionality of certain apparatus described herein such as apparatus 400, 900, 1000), a memory 1328 storing AI model information and/or a database with protocol information.

A third UE 1330 such as a UE comprising a user interface (e.g., smart phone) may comprise at least one of the following modules depicted by Fig. 13: audio input 1332, optical sensor 1334 (e.g., a camera), audio output 1336, an AI model engine 1338 (see above), an NLP model engine 1340 (see above) and/or a motion sensor 1342.

The second and/or third UEs 1316, 1330 may handle the connectivity, from UEs (e.g., using BLE) towards the cloud (e.g., via Wi-Fi or cellular). Local fusion of data, dialog interpretation and 'diagnoses' may be enabled by any of the UEs 1302, 1316, 1330.

Another entity 1344 is implemented in the cloud and comprises at least one of the following module depicted by Fig. 13: a cloud-based AI engine 1346 (e.g., for implementing initial training and further training of the AI model as described above), a cloud-based AI NLP engine 1348 (e.g., for implementing initial training and further training of the NLP model as described above) and a memory 1350 storing information relating to training and using the AI / NLP model. The cloud may comprise certain AI engines, NLP and dialog systems. Cloud-based fusion, interpretation and 'diagnoses' may be enabled in the cloud for certain scenarios which demand more processing power and a learning system to training cloud and edge system AI and NLP engines.

Various embodiments described herein refer to certain models (e.g., AI-based and non-AI based models) for implementing certain functionality such as natural language recognition, understanding context in user dialog, combining information from different sources (e.g., dialog and sensor measurements). Examples of models that may be used to at least partially implement certain embodiments are described below.

Natural language understanding (NLU) and natural language generation (NLG) are concepts used in certain embodiments. These concepts may be referred to as natural language processing (NLP).

Certain embodiments refer to training of a model (e.g., an NLP model or another AI model implementing certain functionality). The learning part may be partly supervised learning (e.g., with the existing protocols, historical data and/or technical 'known' input) and partly unsupervised learning (e.g., with user input such as user dialog, inferred information about the user such as their behavior in relation to the personal care regime).

Speech/dialog recognition and understanding may be implemented using a model that is based on a Recurrent Neural Network (RNN), Time delay Neural network TDNN and/or Deep feedforward Neural Network (DNN).

Automatic speech recognition (e.g., non AI-based) may use Photonic-based algorithms (e.g., the Hidden Markov Model (HHM)) where the algorithm needs to be trained specifically on pronunciations, acoustic and language models.

An example model implementation for understanding speech/dialog may be an intention-based model such as a Listen Attend and Spell (LAS) model. A LAS model may examine an acoustic signal and try and produce a transcript one character at a time. In contrast to certain models, the LAS model may not depend on a conditional model and may be capable of learning all components of the speech, pronunciation, acoustic and language specific models. In such models, there may be no need to deploy large amounts of language model data. LAS may be extended to Watch LAS (WLAS) by adding lip reading functions to better improve speech recognition. In a similar manner, sensor readings and other context could be added to an LAS model to improve context understanding/interpretation of the user input/sensor readings.

In some cases, any of the modules described in relation to any of the embodiments described above may comprise at least one dedicated processor (e.g., an application specific integrated circuit (ASIC) and/or field programmable gate array (FPGA), etc) for implementing the functionality of the module.

In some cases, any of the modules described in relation to any of the embodiments described above may comprise at least one processor for implementing instructions which cause the at least one processor to implement the functionality of the module described above. In such examples, the instructions may be stored in a machine-readable medium (not shown) accessible to the at least one processor. In some examples, the module itself comprises the machine-readable medium. In some examples, the machine-readable medium may be separate to the module itself (e.g., the at least one processor of the module may be provided in communication with the machine readable medium to access the instructions stored therein).

While the invention has been illustrated and described in detail in the drawings and foregoing description, such illustration and description are to be considered illustrative or exemplary and not restrictive; the invention is not limited to the disclosed embodiments.

One or more features described in one part of this disclosure may be combined with or replace features described in another part of this disclosure. For example, the methods 600, 1100 of Figs. 6, 11 may be modified based on features described in relation to the systems 200, 300, 500, the machine-readable media 700, 1200 and/or the apparatus 100, 400, 900, 1000, and vice versa.

This disclosure includes subject-matter defined by the following numbered clauses.
1. An apparatus comprising processing circuitry, the processing circuitry comprising: a determining module configured to interpret natural language in a user data sample associated with use of a personal care device as part of a personal care regime to determine contextual information indicative of a user interaction with the personal care device; and a dialog management module configured to use the contextual information to determine a reaction to the user data sample in accordance with a user protocol stored in a memory accessible to the apparatus.
2. The apparatus of clause 1, wherein the determining module is configured to determine contextual information indicative of the user interaction with the personal care device based on a sensor measurement associated with use of the personal care device as part of the personal care regime.
3. The apparatus of any one of clauses 1 to 2, wherein the dialog management module is configured to generate an indication of the reaction for causing a user interface to interact with a user of the personal care device.
4. The apparatus of clause 3, wherein the indication comprises a message to be issued, via the user interface, to a user of the personal care device and/or a control instruction to control an operation of the personal care device.
5. The apparatus of any one of clauses 1 to 4, wherein the determining module is configured to implement a natural language processing, NLP, model to interpret natural language in the user data sample associated with use of the personal care device as part of the personal care regime and determine the contextual information indicative of the user interaction with the personal care device.
6. The apparatus of any one of clauses 1 to 5, wherein the dialog management module is configured to implement an artificial intelligence, AI, model to determine the reaction to the user data sample in accordance with the user protocol stored in the memory accessible to the apparatus.
7. The apparatus of clause 6, wherein the AI model is configured to personalize the user protocol for a user of the personal care device based on the user data sample.
8. The apparatus of clause 7, wherein the dialog management module is configured to use a previously-obtained protocol stored in the memory to determine a user query based on the user data sample, and wherein the dialog management module is configured to cause a user interface to present the user query at a user interface, and wherein the AI model is configured to personalize the user protocol based on an additional user data sample received in response to the user query.
9. The apparatus of any one of clauses 6 to 8, wherein the AI model is initially trained based on a pre-determined protocol, wherein the pre-determined protocol is based on a user behavioral database comprising information about a plurality of users' interaction with a specified type of personal care device associated with the personal care regime and/or is based on expert input about how users interact with the specified type of personal care device associated with the personal care regime.
10. The apparatus of any one of clauses 1 to 9, wherein the dialog management module is configured to update the user protocol using a dialog exchange between the dialog management module and a user of the personal care device based on the dialog management module making an association between a previous version of the user protocol and contextual information in dialog received from the user.
11. The apparatus of any one of clauses 1 to 10, wherein the dialog management module is configured to update the user protocol by associating a sensor measurement obtained in relation to use of the personal care device by making an association between a previous version of the user protocol and contextual information determined from the sensor measurement.
12. The apparatus of any one of clauses 1 to 11, wherein the processing circuitry comprises: a receiving module configured to receive, from another entity via a communication channel, model information for implementing functionality of the determining module and/or dialog management module.
13. The apparatus of clause 12, wherein the processing circuitry comprises a transmitting module configured to send, in response to the determining module being unable to interpret at least part of the user data sample and/or the dialog management module being unable to determine how to react to at least part of the user data sample, the user data sample for processing by the other entity to determine updated model information for updating the functionality of the determining module to interpret the user data sample and/or updating the functionality of the dialog management module to determine how to react to the user data sample.
14. A computer-implemented method, comprising: interpreting natural language in a user data sample associated with use of a personal care device as part of a personal care regime to determine contextual information indicative of a user interaction with the personal care device; and using the contextual information to determine a reaction to the user data sample in accordance with a user protocol.
15. A tangible machine-readable medium storing instructions which, when executed by at least one processor, cause the at least one processor to implement the method according to clause 14.

Embodiments in the present disclosure can be provided as methods, systems or as a combination of machine-readable instructions and processing circuitry. Such machine-readable instructions may be included on a non-transitory machine (for example, computer) readable storage medium (including but not limited to disc storage, CD-ROM, optical storage, etc.) having computer readable program codes therein or thereon.

The present disclosure is described with reference to flow charts and block diagrams of the method, devices and systems according to embodiments of the present disclosure. Although the flow charts described above show a specific order of execution, the order of execution may differ from that which is depicted. Blocks described in relation to one flow chart may be combined with those of another flow chart. It shall be understood that each block in the flow charts and/or block diagrams, as well as combinations of the blocks in the flow charts and/or block diagrams can be realized by machine readable instructions.

The machine readable instructions may, for example, be executed by a general purpose computer, a special purpose computer, an embedded processor or processors of other programmable data processing devices to realize the functions described in the description and diagrams. In particular, a processor or processing circuitry, or a module thereof, may execute the machine readable instructions. Thus, functional modules of certain apparatus and other devices described herein may be implemented by a processor executing machine readable instructions stored in a memory, or a processor operating in accordance with instructions embedded in logic circuitry. The term 'processor' is to be interpreted broadly to include a CPU, processing unit, ASIC, logic unit, or programmable gate array etc. The methods and functional modules may all be performed by a single processor or divided amongst several processors.

Such machine readable instructions may also be stored in a computer readable storage that can guide the computer or other programmable data processing devices to operate in a specific mode.

Such machine readable instructions may also be loaded onto a computer or other programmable data processing devices, so that the computer or other programmable data processing devices perform a series of operations to produce computer-implemented processing, thus the instructions executed on the computer or other programmable devices realize functions specified by block(s) in the flow charts and/or in the block diagrams.

Further, the teachings herein may be implemented in the form of a computer program product, the computer program product being stored in a storage medium and comprising a plurality of instructions for making a computer device implement the methods recited in the embodiments of the present disclosure.

Elements or steps described in relation to one embodiment may be combined with or replaced by elements or steps described in relation to another embodiment. Other variations to the disclosed embodiments can be understood and effected by those skilled in the art in practising the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. Apparatus (100) comprising processing circuitry (102), the processing circuitry comprising:
a recognition module (104) configured to implement a natural language processing, NLP, model configured to recognize, in a user data sample, a term from a vocabulary associated with a personal care regime, wherein the user data sample comprises a natural language representation of the term;
a generating module (106) configured to generate encoded data indicative of the term, wherein the encoded data uses less memory than the user data sample; and
a transmitting module (108) configured to transmit the encoded data.

2. The apparatus of claim 1, wherein the vocabulary associated with the personal care regime comprises a plurality of terms associated with user interaction with a personal care device for assisting a user with their personal care regime.

3. The apparatus of claim 1 or 2, wherein the NLP model is implemented by an artificial intelligence, AI, model trained to recognize, in the user data sample, the term from the vocabulary associated with the personal care regime.

4. The apparatus of claim 3, wherein the AI-implemented NLP model is trained using user training data derived from a plurality of users' interaction with a specified type of personal care device associated with a specified type of personal care regime.

5. The apparatus of claim 3 or 4, wherein the recognition module is configured to train and/or update the NLP model using the user data sample and/or a sensor measurement associated with a user's personal care regime.

6. The apparatus of any one of claims 1 to 5, wherein the user data sample is in an audio data format, and wherein generated encoded data indicative of the recognized term uses less memory than the user data sample comprising the natural language representation of the recognized term.

7. The apparatus (400) of any one of claims 1 to 6, comprising a memory (404) storing a mapping between at least one term of the vocabulary and corresponding encoded data representative of the at least one term, wherein, in response to recognizing the term in the user data sample, the generating module is configured to access the memory to generate the encoded data corresponding to the recognized term.

8. The apparatus (400) of any one of claims 1 to 7, wherein the processing circuitry (402) comprises a receiving module (406) configured to receive, from another entity, information for implementing functionality of the recognition module and/or generating module.

9. The apparatus of claim 8, wherein the information is received in response to a request sent by the apparatus to the other entity, wherein the request comprises an indication of unrecognizable information in the user data sample and/or a sensor measurement, and the information received in response to the request comprises: an update for the NLP model; at least one term, recognized by the other entity, in the user data sample; and/or contextual information associated with the at least one term recognized by the other entity and/or the sensor measurement.

10. The apparatus of claim 9, wherein the transmitting module is configured to transmit the request in response to the recognition module being unable to recognize at least part of the user data sample and/or interpret the sensor measurement.

11. The apparatus (400) of claim 8, 9 or 10, wherein the received information comprises a response statement generated in response to the term indicated by the encoded data transmitted by the transmitting module, wherein the response statement is in the same data format as the encoded data, the processing circuitry (402) further comprising:
a conversion module (408) configured to convert the response statement to a corresponding natural language representation of the term based on a mapping between the response statement and construction information for constructing the natural language representation of the term.

12. The apparatus of any one of claims 1 to 11, wherein the recognition module is configured to interpret the recognized term from the vocabulary associated with the personal care regime and/or a sensor measurement associated with a user's personal care regime based on a protocol stored in a memory accessible to the recognition module, wherein the protocol specifies a reaction to the interpreted term and/or sensor measurement, and wherein the apparatus is configured to cause a user interface to present the reaction to the user.

13. The apparatus (400) of any one of claims 1 to 12, wherein the processing circuitry (402) comprises a dialog management module (410) configured to:
receive an indication of the recognized term and/or contextual information associated with a user experience; and
generate a response to the indication based on a protocol, wherein the response comprises information for updating the NLP model and/or a request for further information to be collected from the user in accordance with the protocol.

14. A computer-implemented method (600), comprising:
recognizing (602), using a natural language processing, NLP, model, a term from a vocabulary associated with a personal care regime in a user data sample comprising a natural language representation of the term;
generating (604) encoded data indicative of the term, wherein the encoded data uses less memory than the user data sample; and
transmitting (606) the encoded data.

15. A tangible machine-readable medium (700) storing instructions (702) which, when executed by at least one processor (704), cause the at least one processor to implement the method according to claim 14.
